# EUROPÄISCHE PATENTSCHRIFT

(11) **EP 2 536 469 B1**
(45) Veröffentlichungstag und Bekanntmachung des Hinweises auf die Patenterteilung: **19.03.2014**
(21) Anmeldenummer: 11700639.5
(22) Anmeldetag: 11.01.2011
(51) Int. Cl.: A61Q 13/00, C11D 3/50, C11D 3/30, A61K 8/41, A61L 9/01, C11D 7/32

(54) **STABILISIERTE PARFÜMÖLE**
STABILISED PERFUME OILS
HUILES PARFUMÉES STABILISÉES

(30) Priorität: 18.02.2010 DE 102010002106
(43) Veröffentlichungstag der Anmeldung: 26.12.2012
(73) Patentinhaber: Henkel AG & Co. KGaA, 40589 Düsseldorf (DE)
(72) Erfinder: HUCHEL, Ursula, 50670 Köln (DE); GERIGK, Andreas, 41812 Erkelenz (DE); SMYREK, Hubert, 47804 Krefeld (DE); BUNN, Ralf, 40223 Düsseldorf (DE); BAUER, Andreas, 41564 Kaarst (DE); MATERNE, Manuela, 41564 Kaarst (DE); WEYHE, Marc, 47802 Krefeld (DE); INTEMANN, Klaus, 47809 Krefeld (DE); PREIS-AMBERGER, Dagmar, 51375 Leverkusen (DE)
(86) Internationale Anmeldenummer: PCT/EP2011/050252
(87) Internationale Veröffentlichungsnummer: WO 2011/101182

(56) Entgegenhaltungen:
- EP-A1- 1 254 651
- EP-A1- 1 787 689
- DE-A1-102006 017 879
- GB-A- 2 444 702

## Beschreibung

Die vorliegende Erfindung liegt auf dem Gebiet der Parfümzusammensetzungen, wie sie zum Beispiel zur Parfümierung von Verbrauchsprodukten wie z.B. Wasch- oder Reinigungsmittel oder Kosmetika, Verwendung finden oder ganz allgemein zur Erzeugung von Wohlgerüchen einsetzbar sind. Die Erfindung betrifft eine spezielle Parfümzusammensetzung, ein Verfahren zur Stabilisierung von Parfümölen, ein Verfahren zur Herstellung parfümierter Verbrauchsprodukte sowie die Verwendung einer Parfümzusammensetzung bei der Herstellung parfümierter Verbrauchsprodukte.

Ein Problem bei der Handhabung von Parfüms, Riechstoffen und Riechstoffmischungen liegt darin, dass ihr qualitativer Geruchseindruck sowie die Intensität des Geruchs sich gewöhnlich mit der Zeit nachteilig verändern, beispielsweise als Resultat von Umweltbedingungen, z.B. durch Einflüsse von Licht, Wärme, Sauerstoff, Wasser oder Protonen. Diese Instabilität von Parfüms, Riechstoffen und Riechstoffmischungen ist aus offensichtlichen Gründen unerwünscht und es besteht ein kontinuierlicher Bedarf an Parfümen mit verbesserter Stabilität. Dies ist z.B. auch wichtig im Zusammenhang mit dem großtechnischen Einsatz von Parfüms, Riechstoffen und Riechstoffmischungen in der Industrie, bei welcher Parfüms, Riechstoffen und Riechstoffmischungen in großen Mengen gehandhabt werden, lange gelagert werden und in mannigfaltige Produkte eingearbeitet werden.

In DE102006017879 A1 wird beschrieben, Flavonoide, wie z.B. Rutin, als Stabilisatoren für Duftstoffe einzusetzen.

Aufgabe der vorliegenden Erfindung war es daher, besonders stabile Parfümzusammensetzungen hervorzubringen.

Diese Aufgabe wurde gelöst durch eine Parfümzusammensetzung gemäß Anspruch 1, umfassend Aminoalkohol(e) sowie > 10 Gew.-% Riechstoffe. Eine solche Parfümzusammensetzung weist eine sehr gute Lagerstabilität auf und ist robust gegen äußere Einflüsse wie z.B. Wärme.

Neben den Riechstoffen und Aminoalkohol(en) kann die Parfümzusammensetzung vorzugsweise auch noch Hilfsstoffe enthalten, wie insbesondere Lösungsmittel. Geeignete Lösungsmittel sind insbesondere Benzylalkohol, Dipropylengylcol, Dowanol, Ethanol, Isopropanol, Isopropylmyristat, Paraffin, Propylenglycol, Rizinusöl und/oder Triazin. Es können als Hilfsstoffe z.B. auch übliche Stabilisatoren wie z.B. UV.-Absorber (z.B. Benzotriazole), Quencher, Antioxidantien (z.B. gehinderte Phenole, Lacton-Typen und Hydroperoxid-Typen) und Chelatbildner enthalten sein. Andere geeignete Hilfsstoffe sind z.B. Komplexbildner.

Der Aminoalkohol umfasst eine Verbin-dung gemäß Formel (I),

HO-CR1R2-CR3R4-NHR5, (I)

wobei in dieser Formel die Reste R1, R2, R3, R4 sowie R5, jeweils unabhängig voneinander, für Wasserstoff oder (ggf. substituierte) Kohlenwasserstoffreste (insbesondere Hydroxyalkylreste) stehen. Der Kohlenwasserstoffrest kann linear oder verzweigt, substituiert oder unsubstituiert sein.

Besonders bevorzugt ist es, wenn die erfindungsgemäße Parfümzusammensetzung Aminoalkohol gemäß der Formel (II) umfasst 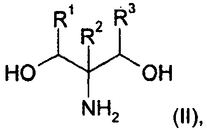 wobei in dieser Formel die Reste R¹, R² sowie R³, jeweils unabhängig voneinander, für Wasserstoff oder (ggf. substituierte) Kohlenwasserstoffreste (insbesondere Hydroxyalkylreste) stehen. Der Kohlenwasserstoffrest kann linear oder verzweigt, substituiert oder unsubstituiert sein.

Besonders bevorzugte Aminoalkohole sind z.B. 2-Aminopropan-1,3-diol, 2-Amino-2-(hydroxymethyl)propan-1,3-diol, 1-(Methylamino)desoxy-D-glucit sowie D-Glucosamin, 2-Amino-2-Methylpropan-1,3-diol, 2-Amino-2-Ethyl-propan-1,3-diol sowie 1-Phenyl-2-Amino-propan-1,3-diol.

Gemäß einer weiteren Ausführungsform der Erfindung ist es möglich, dass der in der erfindungsgemäßen Parfümzusammensetzung enthaltene Aminoalkohol polymergebunden ist, d.h. in der Formel (I) wäre dann zumindest einer der Reste R1, R2, R3, R4, R5 ein polymerer Kohlenwasserstoffrest, bzw. in der Formel (II) wäre zumindest einer der Reste R1, R2, R3 ein polymerer Kohlenwasserstoffrest.

Es ist im Sinne der vorliegenden Erfindung besonders bevorzugt, wenn in der erfindungsgemäßen Parfümzusammensetzung ein hoher Riechstoffgehalt realisiert wird, so dass diese vorteilhafterweise > 15 Gew.-%, > 20 Gew.-%, > 25 Gew.-%, > 30 Gew.-%, > 35 Gew.-%, > 40 Gew.-%, > 45 Gew.-%, > 50 Gew.-%, > 55 Gew.-% oder > 60 Gew.-%, vorzugsweise > 70 Gew.-%, vorteilhafterweise > 80 Gew.-%, insbesondere > 90 Gew.-%, z.B. > 95 Gew.-% Riechstoffe enthält.

Besonders instabil sind in üblichen Riechstoffmischungen solche Riechstoffe, die reaktive funktionelle Gruppen tragen, wie z.B. Aldehyde, Ketone, Alkohole und Amine. Daher werden solche Riechstoffe oftmals nur in untergeordneten Mengen eingesetzt. Unsere Erfindung ermöglicht demgegenüber auch den Einsatz von Riechstoff-Aldehyden, -Ketonen, -Alkoholen und -Aminen in größeren Mengen, ohne Qualitätseinbußen, z.B. bei längerer Lagerung hinnehmen zu müssen. Entsprechend sind gemäß einer bevorzugten Ausführungsform der Erfindung mehr als 1 Gew.-%, in vorteilhafter Weise mehr als 5 Gew.-%, insbesondere mehr als 10 Gew.-%, z.B. mehr als 15, 20 oder 25 Gew.-% der enthaltenen Riechstoffe aus solchen Riechstoffen ausgewählt, welche eine Aldehydfunktion ( RCH=O), eine Keto-Gruppe ( RR'C=O ), eine Hydroxyfunktion (-OH) und/oder Gruppen mit isolierten Doppelbindungen tragen, wobei sich die Angabe Gew.-% auf die Gesamtmenge der enthaltenen Riechstoffe bezieht.

Gemäß einer weiteren bevorzugten Ausführungsform der Erfindung zeichnet sich eine erfindungsgemäße Parfümzusammensetzung dadurch aus, dass als Riechstoff zumindest ein Duftaldehyd enthalten ist, ausgewählt aus Adoxal, Anisaldehyd, Cymal, Ethylvanillin, Florhydral, Helional, Heliotropin, Hydroxycitronellal, Koavon, Lauraldehyd, Lyral, Methylnonylacetaldehyd, P. T. Bucinal, Phenylacetaldehyd, Undecylenaldehyd, Vanillin, 2,6,10-Trimethyl-9-undecenal, 3-Dodecen-1-al, alpha-n-Amylzimtaldehyd, 4-Methoxybenzaldehyd, Benzaldehyd, 3-(4-tert-Butylphenyl)-propanal, 2-Methyl-3-(para-methoxyphenyl)propanal, 2-Methyl-4-(2,6,6-timethyl-2(1)-cyclohexen-1-yl)butanal, 3-Phenyl-2-propenal, cis-/trans-3,7-Dimethyl- 2,6-octadien-1-al, 3,7-Dimethyl-6-octen-1-al, [(3,7-Dimethyl-6-octenyl)oxy]acetaldehyd, 4-Isopropylbenzylaldehyd, 1,2,3,4,5,6,7,8-Octahydro-8,8-dmethyl-2-naphthaldehyd, 2,4-Dimethyl-3-cyclohexen-1-carboxaldehyd, 2-Methyl-3-(isopropylphenyl)propanal, 1-Decanal, Decylaldehyd, 2,6-Dimethyl-5-heptenal, 4-(Tricyclo[ 5.2.1.0(2,6)]-decyliden-8)-butanal, Octahydro-4,7-methan-1H-indencarboxaldehyd, 3-Ethoxy-4-hydroxy-benzaldehyd, para-Ethyl-alpha,alphadimethylhydrozimtaldehyd, alpha-Methyl-3,4-(methylendioxy)-hydrozimtaldehyd, 3,4-Methylendioxybenzaldehyd, alpha-n-Hexylzimtaldehyd, m-Cymen-7-carboxaldehyd, alpha-Methylphenylacetaldehyd, 7-Hydroxy-3,7-dimethyloctanal, Undecenal, 2,4,6-Trimethyl-3-cyclohexen-1-carboxaldehyd, 4-(3)(4-Methyl-3-pentenyl)-3-cyclohexencarboxaldehyd, 1-Dodecanal, 2,4-Dimethylcyclohexen-3-carboxaldehyd, 4-(4-Hydroxy-4-methylpentyl)-3-cylohexen-1-carboxaldehyd, 7-Methoxy-3,7-dimethyloctan-1-al 2-Methylundecanal, 2-Methyldecanal, 1-Nonanal, 1-Octanal, 2,6,10-Trimethyl-5,9-undecadienal, 2-Methyl- 3-(4-tert-butyl)propanal, Dihydrozimtaldehyd, 1-Methyl-4-(4-methyl-3-pentenyl)-3-cyclohexen-1-carboxaldehyd, 5- oder 6-Methoxyhexahydro-4,7-methanindan-1-oder-2-carboxaldehyd, 3,7-Dimethyloctan-1-al, 1-Undecanal, 10-Undecen-1-al, 4-Hydroxy-3-methoxybenzaldehyd, 1-Methyl-3-(4-methylpentyl)-3-cyclohexencarbox-aldehyd, 7-Hydroxy-3,7-dimethyl-octanal, trans-4-Decenal, 2,6-Nonadienal, para-Tolylacetaldehyd, 4-Methylphenylacetaldehyd, 2-Methyl-4-(2,6,6-trimethyl-1-cyclohexen-1-yl)-2-butenal, ortho-Methoxyzimtaldehyd, 3,5,6-Trimethyl-3-cyclohexencarboxaldehyd, 3,7-Dimethyl-2-methylen-6-octenal, Phenoxyacetaldehyd, 5,9-Dimethyl-4,8-decadienal, Päonienaldehyd (6,10-Dimethyl-3-oxa-5,9-undecadien-1-al), Hexahydro- 4,7-methanindan-1-carboxaldehyd, 2-Methyloctanal, alpha-Methyl-4-(1-methylethyl)benzolacetaldehyd, 6,6-Dimethyl-2-norpinen-2-propionaldehyd, para-Methylphenoxyacetaldehyd, 2-Methyl-3-phenyl-2-propen- 1-al 3,5,5-Trimethylhexanal, Hexahydro-8,8-dimethyl-2-naphthaldehyd, 3-Propylbicyclo[ 2.2.1]-hept-5-en-2-carbaldehyd, 9-Decenal, 3-Methyl-5-phenyl-1-pentanal, Methylnonylacetaldehyd, Hexanal, trans-2-Hexenal, 1-p-Menthen-q-carboxaldehyd oder Mischungen davon. Vorzugsweise sind zumindest 2, 3, 4 oder 5 der zuvor genannten Duftaldehyde enthalten. Vorzugsweise sind gemäß einer bevorzugten Ausführungsform der Erfindung mehr als 1 Gew.-%, in vorteilhafter Weise mehr als 5 Gew:-%, insbesondere mehr als 10 Gew.-%, z.B. mehr als 15, 20 oder 25 Gew.-% der in der Parfümzusammensetzung enthaltenen Riechstoffe Duftaldehyde, insbesondere solche wie zuvor genannt.

Wenn als Riechstoff zumindest ein Duftketon in der erfindungsgemäßen Parfümzusammensetzung enthalten ist, ausgewählt aus Buccoxim; iso-Jasmon; Methyl-beta-naphthylketon; Moschusindanon; Tona-lid/Musk plus; alpha-Damascon, beta-Damascon, delta-Damascon, iso-Damascon, Damascenon, Damarose, Methyldihydrojasmonat, Menthon, Carvon, Kampfer, Fenchon, alpha-lonon, beta-Ionon, gamma-Methyl ge-nannt Ionon, Fleuramon, Dihydrojasmon, cis-Jasmon, iso-E-Super^{®}, Methylcedrenylketon oder Methylcedry-Ion, Acetophenon, Methylacetophenon, para-Methoxyacetophenon, Methyl-beta-naphtylketon, Benzylaceton, Benzophenon, para-Hydroxyphenylbutanon, Sellerie-Keton oder Livescone, 6-Isopropyldecahydro-2-naphton, Dimethyloctenon, Frescomenthe, 4-(1-Ethoxyvinyl)-3,3,5,5-tetramethylcyclohexanon, Methylheptenon, 2-(2-(4-Methyl-3-cyclohexen-1-yl)propyl)cyclopentanon, 1-(p-Menthen-6(2)yl)-1-propanon, 4-(4-Hydro-xy-3-methoxyphenyl)-2-butanon, 2-Acetyl-3,3-dimethylnorbornan, 6,7-Dihydro-1,1,2,3,3-pentamethyl-4(5H)indanon, 4-Damascol, Dulcinyl oder Cassion, Gelson, Hexalon, Isocyclemon E, Methylcyclocitron, Methyllaven-delketon, Orivon, para-tertiärem Butylcyclohexanon, Verdon, Delphon, Muscon, Neobutenon, Plicaton, Velou-ton, 2,4,4,7-Tetramethyl-oct-6-en-3-on, Tetrameran oder Mischungen davon so liegt ebenfalls eine bevorzugten Ausführungsform der Erfindung vor. Vorzugsweise sind zumindest 2, 3, 4 oder 5 der zuvor genannten Duftketone enthalten. Vorzugsweise sind gemäß einer bevorzugten Ausführungsform der Erfindung mehr als 1 Gew.-%, in vorteilhafter Weise mehr als 5 Gew.-%, insbesondere mehr als 10 Gew.-%, z.B. mehr als 15, 20 oder 25 Gew.-% der in der Parfümzusammensetzung enthaltenen Riechstoffe Duftketone, insbesondere solche wie zuvor genannt.

Weiterhin entspricht es einer bevorzugten Ausführungsform der Erfindung, wenn Aminoalkohol in Mengen von 0,001 bis 30 Gew.-%, vorzugsweise 0,1 bis 10 Gew.-%, insbesondere 1 bis 5 Gew.-% in der Parfümzusammensetzung enthalten ist. Geeignete Untergrenzen für den Aminoalkohol können auch bei 0,5 Gew.-%, 1,5 Gew.-% oder 2 Gew.-% liegen. Geeignete Obergrenzen für den Aminoalkohol können auch bei 25 Gew.-%, 20 Gew.-% oder 15 Gew.-% liegen.

Die erfindungsgemäße Parfümzusammensetzung kann grundsätzlich alle üblichen und bekannten Riechstoffe enthalten. Riechstoffe sind dem Fachmann in breiter Vielfalt bekannt, es kann sich bei den enthaltenen Riechstoffen z.B. um Riechstoffe der folgenden Verbindungsklassen handeln: Terpenoide, Brenzcatechin-Derivate, Phenol-Derivate, sonstige Aromaten, Aliphaten, Alicyclen und Heterocyclen.

Nach einer weiteren bevorzugten Ausführungsform können in der erfindungsgemäßen Parfümzusammensetzung z.B. Riechstoffe mit Duftnoten der Grün-Noten (wie z.B. Hex-3-en-1-ol), Zitrus-Noten (wie z.B. Citral), Lavendel-Noten (wie z.B. Lavandinöl), Blumige-Noten (wie z.B. Geraniol, Jonon), Aldehyd-Noten (wie z.B. Alkanale und Alken-1-ale C8-C13), Chypre-Noten (wie z.B. Labdanum), Fougere-Noten (wie z.B. Cumarin), Gewürz-Noten (wie z.B. Eugenol), Orientalischeund/oder Holz-Noten (wie z.B. Santalol, Cedrol) und/oder Leder bzw. Tabak-Noten (wie z.B. Muscon, Ambra-Oxid, Zibet) enthalten sein.

Nach einer weiteren bevorzugten Ausführungsform können z.B. Riechstoffe aus der Gruppe umfassend Riechstoffe natürlichen oder synthetischen Ursprungs, bevorzugt leichter flüchtige Riechstoffe, höhersiedende Riechstoffe, feste Riechstoffe und/oder haftfeste Riechstoffe enthalten sein.

Es ist ein zusätzlicher Vorteil der Erfindung, dass die erfindungsgemäße Parfümzusammensetzung einen duftverstärkenden Effekt hervorruft, d.h. der Dufteindruck am bedufteten Objekt wird intensiver und er hält länger an, insbesondere bei Anwendung im Zusammenhang mit der Textilwäsche oder -Pflege.

Haftfeste Riechstoffe, die im Rahmen der vorliegenden Erfindung einsetzbar sind, sind beispielsweise etherische Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaivabalsamöl, Korianderöl, Krauseminzeöl, Kümmelöl, Kuminöl, Lavendelöl, Lemon-grasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl.

Aber auch höhersiedende bzw. feste Riechstoffe natürlichen oder synthetischen Ursprungs können im Rahmen der vorliegenden Erfindung eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, α-Bromstyrol, n-Decylaldehyd, n-Dodecylalde-hyd, Eugenof, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon- Dimethylether, Hydroxyzimtaldehyd, Hydroxyzimtalkohol, Indol, Iron, Isoeugenol, Isoeuge-nolmethylether, Isosafrol, Jasmon, Kampfer, Karvakrol, Karvon, p-Kresol-methylether, Cumarin, p-Methoxyacetophenon, Methyl-n-amylketon, Methylanthranil-säure-methylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphtholmethylether, Nerol, Nitrobenzol, n-Nonylaldehyd, Nonylakohol, n-Octylaldehyd, p-Oxy-Acetophenon, Pentadekanolid, β-Phenylethylalkohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanilin, Veratrumaldehyd, Zimtaldehyd, Zimatalkohol, Zimtsäure, Zimtsäureethylester, Zimtsäurebenzylester.

Zu den leichter flüchtigen Riechstoffen zählen insbesondere die niedriger siedenden Riechstoffe natürlichen oder synthetischen Ursprung, die allein oder in Mischungen eingesetzt werden können. Beispiele für leichter flüchtige Riechstoffe sind Alkyisothiocyanate (Alkylsenföle), Butandion, Limonen, Linalool, Linaylacetat und -Propionat, Menthol, Menthon, Methyl-n-heptenon, Phellandren, Phenylacetaldehyd, Terpinylacetat, Zitral, Zitronellal.

Nach einer weiteren bevorzugten Ausführungsform der Erfindung zeichnet sich die erfindungsgemäße Parfümzusammensetzung dadurch aus, dass diese ≤ 15 Gew.-%, vorzugsweise ≤ 5 Gew.-%, insbesondere ≤ 1 Gew.-% Tenside umfasst. Der Tensidgehalt kann auch unter 10 Gew.-% oder unter 2 Gew.-% oder unter 0,5 Gew.-% oder unter 0,1 Gew.-% oder unter 0,01 Gew.-% liegen.

Wenn Tenside enthalten sind, was optional ist, dann kann eine geeignete Mindestmenge bei 0,001 Gew.-% liegen, Gew.-% jeweils bezogen auf die gesamte Zusammensetzung. Unter den Begriff der Tenside fallen im Sinne der Erfindung auch die Emulgatoren als grenzflächenaktive Stoffe. Bevorzugt einsetzbare Emulgatoren sind ethoxylierte Fettalkohole, z.B. C12-18-Fettalkohol mit 5 EO (z.B. Dehydol LT 5 ex Cognis), ethoxylierte Triglyceride (z.B. Eumulgin HRE 40, Eumulgin HRE 60 ex Cognis), Sorbitanfettsäureester, z.B. Polyoxyethylensorbitanfettsäurester, z.B. Polyoxyethylensorbitanmonolaurat (z.B. Tween 20 ex Merck) sowie hydriertes, ethoxyliertes Rizinusöl (z.B. Cremophor RH 40 ex BASF).

Besonders gute erfindungsgemäße Resultate lassen sich erzielen, wenn die erfindungsgemäße Parfümzusammensetzung zu ≥ 50 Gew.-%, vorzugsweise ≥ 70 Gew.-%, insbesondere ≥ 80 Gew.-% (wie z.B. ≥ 90, ≥ 95, ≥ 97 oder ≥ 99 Gew.-%) aus den Bestandteilen Parfüm, Lösungsmittel und Aminoalkohol besteht.

Besonders gute erfindungsgemäße Resultate lassen sich auch erzielen, wenn die erfindungsgemäße Parfümzusammensetzung zu ≥ 50 Gew.-%, vorzugsweise ≥ 70 Gew.-%, insbesondere ≥ 80 Gew.-%, z.B. ≥ 90 Gew.-% oder ≥ 95 Gew.-% aus den Bestandteilen Parfüm und Aminoalkohol besteht.

Die erfindungsgemäße Parfümzusammensetzung ist vorzugsweise flüssig oder gelartig. Dies entspricht einer bevorzugten Ausführungsform der Erfindung. Sie kann aber auch fest sein, aber flüssige oder gelartige Ausführungsformen sind deutlich bevorzugt.

Ein weiterer Gegenstand der Erfindung ist ein Verfahren zur Stabilisierung von Parfümölen, bei welchem 0,001 bis 30 Gew.-% Aminoalkohol gemäß Formel (I) (vorzugsweise 0,1 bis 10 Gew.-%, insbesondere 1 bis 5 Gew.-%) in das Parfümöl gegeben werden. Unabhängig von der Natur des Parfümöls, d.h. seiner stoffliche Zusammensetzung, bewirkt die Zugabe von Aminoalkohol zum Parfümöl dessen Stabilisierung und bewirkt so z.B. eine verbesserte Lagerstabilität sowie eine verbesserte Robustheit gegenüber äußeren Einflüssen, wie z.B. Wärme, Luft, Verunreinigungen. Bevorzugte Aminoalkohole wurden bereits genannt.

Ein weiterer Gegenstand der Erfindung ist ein Verfahren zur Herstellung parfümierter Verbrauchsprodukte (insbesondere Wasch- oder Reinigungsmittel, Kosmetika, Luftverbesserer, Klebstoffe), wobei eine erfindungsgemäße Parfümzusammensetzung zugegeben wird. Die resultierenden Verbrauchsprodukte entfalten eine überlegene Duftwirkung bei der Anwendung, sie zeichnen sich durch eine besonders gute Lagerstabilität aus.

Ein weiterer Gegenstand der Erfindung ist somit die Verwendung einer erfindungsgemäßen Parfümzusammensetzung bei der Herstellung parfümierter Verbrauchsprodukte zur Verbesserung der Lagerstabilität des Verbrauchsproduktes.

Die Verwendung einer erfindungsgemäßen Parfümzusammensetzung bei der Herstellung parfümierter Wasch-, Reinigungs- oder Pflegemittels zur Verlängerung der Duftwirkung des Wasch-, Reinigungs- oder Pflegemittels und/oder zur Erzielung eines lange anhaltenden Frischegeruches bei der Anwendung des Wasch-, Reinigungs- oder Pflegemittels (insbesondere mit Blick auf trockene Textilien nach der maschinellen Textilwäsche) wird auch offenbart.

Verbrauchsprodukte, die durch Einarbeitung der erfindungsgemäßen Parfümzusammensetzung von unserer Erfindung profitieren sind insbesondere Wasch- oder Reinigungsmittel, Kosmetika, Luftverbesserer sowie Klebstoffe. Hier gelingen stabilere Produkte.

Bevorzugt enthalten solche Verbrauchsprodukte, wie insbesondere Wasch, Pflege- oder Reinigungsmittel, wenigstens eine, vorzugsweise mehrere, aktive Komponenten, insbesondere wasch-, pflege-, reinigungsaktive und/oder kosmetische Komponenten, vorteilhafterweise ausgewählt aus der Gruppe umfassend anionische Tenside, kationische Tenside, amphotere Tenside, nichtionische Tenside, Acidifizierungsmittel, Alkalisierungsmittel, Anti-Knitter-Verbindungen, antibakterielle Stoffe, Antioxidantien, Antiredepositionsmittel, Antistatika, Buildersubstanzen, Bleichmittel, Bleichaktivatoren, Bleichstabilisatoren, Bleichkatalysatoren, Bügelhilfsmittel, Cobuilder, Riechstoffe, Einlaufverhinderer, Elektrolyte, Enzyme, Farbschutzstoffe, Färbemittel, Farbstoffe, Farbübertragungsinhibitoren, Fluoreszensmittel, Fungizide, Germizide, geruchskomplexierende Substanzen, Hilfsmittel, Hydrotrope, Klarspüler, Komplexbildner, Konservierungsmittel, Korrosionsinhibitoren, optische Aufheller, Parfüme, Parfümträger, Perlglanzgeber, pH-Stellmittel, Phobier- und Imprägniermittel, Polymere, Quell- und Schiebefestmittel, Schauminhibitoren, Schichtsilikate, schmutzabweisende Stoffe, Silberschutzmittel, Silikonöle, UV-Schutz-Substanzen, Viskositätsregulatoren, Verdickungsmittel, Verfärbungsinhibitoren, Vergrauungsinhibitoren, Vitamine und/oder Weichspüler.

Die Mengen der einzelnen Inhaltsstoffe in den erfindungsgemäßen Verbrauchsprodukten, wie insbesondere Wasch, Pflege- oder Reinigungsmitteln, orientieren sich jeweils am Einsatzzweck der betreffenden Mittel und der Fachmann ist mit den Größenordnungen der einzusetzenden Mengen der Inhaltsstoffe vertraut oder kann diese der zugehörigen Fachliteratur entnehmen.

Je nach Einsatzzweck der erfindungsgemäßen Verbrauchsprodukte, wie insbesondere Wasch, Pflege- oder Reinigungsmittel, wird man beispielsweise den Tensidgehalt höher oder niedriger wählen. Üblicherweise liegt z. B. der Tensidgehalt beispielsweise von Waschmitteln zwischen 10 und 50 Gew.-%, vorzugsweise zwischen 12,5 und 30 Gew.-% und insbesondere zwischen 15 und 25 Gew.-%, während Reinigungsmittel für das maschinelle Geschirrspülen üblicherweise zwischen 0,1 und 10 Gew.-%, vorzugsweise zwischen 0,5 und 7,5 Gew.-% und insbesondere zwischen 1 und 5 Gew.-% Tenside enthalten.

Erfindungsgemäße Verbrauchsprodukte können insbesondere Buildersubstanzen, oberflächenaktive Tenside, Enzyme, Bleichmittel, wie vorzugsweise organische und/oder anorganische Persauerstoffverbindungen, Persauerstoff-Aktivatoren, wassermischbare organische Lösungsmittel, Sequestrierungsmittel, Elektrolyte, pH-Regulatoren, Verdicker und weitere Hilfsstoffe, wie soil release-Wirkstoffe, optische Aufheller, Vergrauungsinhibitoren, Farbübertragungsinhibitoren, Schaumregulatoren sowie Farbstoffe enthalten.

Die erfindungsgemäßen Verbrauchsprodukte können Tenside enthalten, wobei insbesondere anionische Tenside, nichtionische Tenside und deren Gemische, aber auch kationische Tenside in Frage kommen. Geeignete nichtionische Tenside sind insbesondere Ethoxylierungs- und/oder Propoxylierungsprodukte von Alkylglykosiden und/oder linearen oder verzweigten Alkoholen mit jeweils 12 bis 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkylaminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar.

Geeignete anionische Tenside sind insbesondere Seifen und solche, die Sulfat- oder SulfonatGruppen mit bevorzugt Alkaliionen als Kationen enthalten. Verwendbare Seifen sind bevorzugt die Alkalisalze der gesättigten oder ungesättigten Fettsäuren mit 12 bis 18 C-Atomen. Derartige Fettsäuren können auch in nicht vollständig neutralisierter Form eingesetzt werden. Zu den brauchbaren Tensiden des Sulfat-Typs gehören die Salze der Schwefelsäurehalbester von Fettalkoholen mit 12 bis 18 C-Atomen und die Sulfatierungsprodukte der genannten nichtionischen Tenside mit niedrigem Ethoxylierungsgrad. Zu den verwendbaren Tensiden vom Sulfonat-Typ gehören lineare Alkylbenzolsulfonate mit 9 bis 14 C-Atomen im Alkylteil, Alkansulfonate mit 12 bis 18 C-Atomen, sowie Olefinsulfonate mit 12 bis 18 C-Atomen, die bei der Umsetzung entsprechender Monoolefine mit Schwefeltrioxid entstehen, sowie alpha-Sulfofettsäureester, die bei der Sulfonierung von Fettsäuremethyl- oder -ethylestern entstehen.

Kationische Tenside werden vorzugsweise unter den Esterquats und/oder den quaternären Ammoniumverbindungen (QAV) gemäß der allgemeinen Formel (R^{I})(R^{II})(R^{III})(R^{IV})N⁺ X⁻ ausgewählt, in der R^{I} bis R^{IV} für gleiche oder verschiedene C₁₋₂₂-Alkylreste, C₇₋₂₈-Arylalkylreste oder heterozyklische Reste stehen, wobei zwei oder im Falle einer aromatischen Einbindung wie im Pyridin sogar drei Reste gemeinsam mit dem Stickstoffatom den Heterozyklus, z.B. eine Pyridinium- oder Imidazoliniumverbindung, bilden, und X⁻ für Halogenidionen, Sulfationen, Hydroxidionen oder ähnliche Anionen steht. QAV sind durch Umsetzung tertiärer Amine mit Alkylierungsmitteln, wie z.B. Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer MethylGruppe kann mit Hilfe von Methylchlorid unter milden Bedingungen durchgeführt werden. Amine, die über drei lange Alkyl-Reste oder Hydroxy-substituierte Alkyl-Reste verfügen, sind wenig reaktiv und werden beispielsweise mit Dimethylsulfat quaterniert. In Frage kommende QAV sind beispielweise Benzalkoniumchlorid (N Alkyl-N,N dimethyl-benzylammoniumchlorid), Benzalkon B (m,p-Dichlorbenzyl-dimethyl-C₁₂-alkylammoniumchlorid, Benzoxoniumchlorid (Benzyl-dodecyl-bis-(2-hydroxyethyl)-ammoniumchlorid), Cetrimoniumbromid (N-Hexadecyl-N,N-trimethyl-ammoniumbromid), Benzetoniumchlorid (N,N Dimethyl-N [2-[2-[p-(1,1,3,3-tetramethylbutyl)phenoxy]-ethoxy]-ethyl]-benzylammoniumchlorid), Dialkyldimethylammoniumchloride wie Di-n-decyl-dimethyl-ammoniumchlorid, Didecyldimethylammonium-bromid, Dioctyl-dimethyl-ammoniumchlorid, 1-Cetylpyridiniumchlorid und Thiazolinjodid sowie deren Mischungen. Bevorzugte QAV sind die Benzalkoniumchloride mit C₈-C₂₂-Alkylresten, insbesondere C₁₂-C₁₄-Alkyl-benzyldimethylammoniumchlorid.

Unter Esterquats sollen hier vorzugsweise Verbindungen der allgemeinen Formel IV, 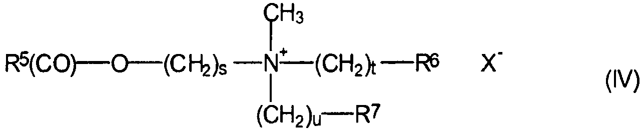 verstanden werden, in der R⁵ für einen Alkyl- oder Alkenylrest mit 12 bis 22 Kohlenstoffatomen und 0, 1, 2 oder 3 Doppelbindungen, R⁶ und R⁷ unabhängig voneinander für H, OH oder O(CO)R⁵, s, t und u jeweils unabhängig voneinander für den Wert 1, 2 oder 3 und X⁻ für ein Anion, insbesondere Halogenid, Methosulfat, Methophosphat oder Phosphat sowie Mischungen aus diesen, steht. Bevorzugt sind Verbindungen, die für R⁶ die Gruppe O(CO)R⁵ und für R⁵ einen Alkylrest mit 16 bis 18 Kohlenstoffatomen enthalten. Besonders bevorzugt sind Verbindungen, bei denen R⁷ zudem für OH steht. Beispiele für Verbindungen der Formel (IV) sind Methyl-N-(2-hydroxyethyl)-N,N-di(talgacyl-oxyethyl)ammonium-methosulfat, Bis-(palmitoyl)-ethyl-hydroxyethyl-methyl-ammonium-methosulfat oder Methyl-N,N-bis(acyloxyethyl)-N-(2-hydroxyethyl)ammonium-methosulfat. Werden quarternierte Verbindungen der Formel (IV) eingesetzt, die ungesättigte Gruppen aufweisen, sind die Acylgruppen bevorzugt, deren korrespondierende Fettsäuren eine Jodzahl zwischen 5 und 80, vorzugsweise zwischen 10 und 60 und insbesondere zwischen 15 und 45 aufweisen und/oder die ein cis/trans-Isomerenverhältnis (in Mol-%) von größer als 30 : 70, vorzugsweise größer als 50 : 50 und insbesondere größer als 70 : 30 haben. Handelsübliche Beispiele sind die von der Firma Stepan unter dem Warenzeichen Stepantex^{®} vertriebenen Methylhydroxyalkyldialkoyloxyalkylammoniummethosulfate oder die unter dem Handelsnamen Dehyquart^{®} bekannten Produkte der Firma Cognis Deutschland GmbH beziehungsweise die unter der Bezeichnung Rewoquat^{®} bekannten Produkte des Herstellers Goldschmidt-Witco.

Tenside sind in erfindungsgemäßen Verbrauchsprodukten, insbesondere Waschmitteln, in Mengenanteilen von vorzugsweise 5 Gew.-% bis 50 Gew.-%, insbesondere von 8 Gew.-% bis 30 Gew.-%, enthalten. Insbesondere in Wäschenachbehandlungsmittein werden vorzugsweise bis zu 30 Gew.-%, insbesondere 5 Gew.-% bis 15 Gew.-% Tenside, unter diesen bevorzugt wenigstens anteilsweise Kationtenside, eingesetzt.

Ein erfindungsgemäßes Verbrauchsprodukt enthält vorzugsweise mindestens einen wasserlöslichen und/oder wasserunlöslichen, organischen und/oder anorganischen Builder. Zu den wasserlöslichen organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphonsäuren, insbesondere Aminotris(methylenphosphonsäure), Ethylendiamintetrakis(methylenphosphonsäure) und 1-Hydroxyethan-1,1-diphosphonsäure, polymere Hydroxyverbindungen wie Dextrin sowie polymere (Poly-)carbonsäuren, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Die relative Molekülmasse der Homopolymeren ungesättiger Carbonsäuren liegt im allgemeinen zwischen 5 000 und 200 000, die der Copolymeren zwischen 2 000 und 200 000, vorzugsweise 50 000 bis 120 000, jeweils bezogen auf freie Säure. Ein besonders bevorzugtes Acrylsäure-Maleinsäure-Copolymer weist eine relative Molekülmasse von 50 000 bis 100 000 auf. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt.

Organische Buildersubstanzen können gewünschtenfalls in Mengen bis zu 40 Gew.-%, insbesondere bis zu 25 Gew.-% und vorzugsweise von 1 Gew.-% bis 8 Gew.-% in den erfindungsgemäßen Verbrauchsprodukten enthalten sein. Mengen nahe der genannten Obergrenze werden vorzugsweise in pastenförmigen oder flüssigen, insbesondere wasserhaltigen, erfindungsgemäßen Verbrauchsprodukten eingesetzt. Erfindungsgemäße Verbrauchsprodukte wie Wäschenachbehandlungsmittel, wie z.B. Weichspüler, können gegebenenfalls auch frei von organischem Builder sein.

Als wasserlösliche anorganische Buildermaterialien kommen insbesondere Alkalisilikate und Polyphosphate, vorzugsweise Natriumtriphosphat, in Betracht. Als wasserunlösliche, wasserdispergierbare anorganische Buildermaterialien werden insbesondere kristalline oder amorphe Alkalialumosilikate, in Mengen von z.B. bis zu 50 Gew.-%, vorzugsweise nicht über 40 Gew.-% und in flüssigen Mitteln insbesondere von 1 Gew.-% bis 5 Gew.-%, in den erfindungsgemäßen Verbrauchsprodukten eingesetzt. Unter diesen sind die kristallinen Natriumalumosilikate in Waschmittelqualität, insbesondere Zeolith A, P und gegebenenfalls X, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm.

Geeignete Substitute beziehungsweise Teilsubstitute für das genannte Alumosilikat sind kristalline Alkalisilikate, die allein oder im Gemisch mit amorphen Silikaten vorliegen können. Die in den erfindungsgemäßen Verbrauchsprodukten als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen.

Buildersubstanzen sind in den erfindungsgemäßen Verbrauchsprodukten vorzugsweise in Mengen bis zu 60 Gew.-%, insbesondere von 5 Gew.-% bis 40 Gew.-%, enthalten. Erfindungsgemäße Wäschenachbehandlungsmittel, wie z.B. Weichspüler, sind vorzugsweise frei von anorganischem Builder.

Als geeignete Persauerstoffverbindungen kommen insbesondere organische Persäuren beziehungsweise persaure Salze organischer Säuren, wie Phthalimidopercapronsäure, Perbenzoesäure oder Salze der Diperdodecandisäure, Wasserstoffperoxid und unter den Anwendungsbedingungen Wasserstoffperoxid abgebende anorganische Salze, wie Perborat, Percarbonat und/oder Persilikat, in Betracht. Sofern feste Persauerstoffverbindungen eingesetzt werden sollen, können diese in Form von Pulvern oder Granulaten verwendet werden, die auch in im Prinzip bekannter Weise umhüllt sein können. Besonders bevorzugt wird Alkalipercarbonat, Alkaliperborat-Monohydrat oder insbesondere in flüssigen Mitteln Wasserstoffperoxid in Form wäßriger Lösungen, die 3 Gew.-% bis 10 Gew.-% Wasserstoffperoxid enthalten, eingesetzt. Falls ein erfindungsgemäßes Verbrauchsprodukt Bleichmittel, wie vorzugsweise Persauerstoffverbindungen, enthält, sind diese in Mengen von vorzugsweise bis zu 50 Gew.-%, insbesondere von 5 Gew.-% bis 30 Gew.-%, vorhanden. Der Zusatz geringer Mengen bekannter Bleichmittelstabilisatoren wie beispielsweise von Phosphönaten, Boraten beziehungsweise Metaboraten und Metasilikaten sowie Magnesiumsalzen wie Magnesiumsulfat kann zweckdienlich sein.

Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofuran und Enolester sowie acetyliertes Sorbitol und Mannitol beziehungsweise deren Mischungen (SORMAN), acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Gluconolacton, und/oder N-acylierte Lactame, beispielsweise N-Benzoylcaprolactam. Hydrophil substituierte Acylacetale und Acyllactame werden ebenfalls bevorzugt eingesetzt. Auch Kombinationen konventioneller Bleichaktivatoren können eingesetzt werden. Derartige Bleichaktivatoren können im üblichen Mengenbereich, vorzugsweise in Mengen von 1 Gew.-% bis 10 Gew.-%, insbesondere 2 Gew.-% bis 8 Gew.-%, bezogen auf gesamtes Verbrauchsprodukt, enthalten sein.

Als in den Verbrauchsprodukten verwendbare Enzyme kommen solche aus der Klasse der Proteasen, Cutinasen, Amylasen, Pullulanasen, Hemicellulasen, Cellulasen, Lipasen, Oxidasen und Peroxidasen sowie deren Gemische in Frage. Besonders geeignet sind aus Pilzen oder Bakterien, wie Bacillus subtilis, Bacillus licheniformis, Streptomyces griseus, Humicola lanuginosa, Humicola insolens, Pseudomonas pseudoalcaligenes oder Pseudomonas cepacia gewonnene enzymatische Wirkstoffe. Die gegebenenfalls verwendeten Enzyme können an Trägerstoffen adsorbiert und/oder in Hüllsubstanzen eingebettet sein, um sie gegen vorzeitige Inaktivierung zu schützen. Sie sind in den erfindungsgemäßen Verbrauchsprodukten, insbesondere Waschmitteln, vorzugsweise nicht über 5 Gew.-%, insbesondere von 0,2 Gew.-% bis 2 Gew.-%, enthalten.

Die Verbrauchsprodukte können als optische Aufheller beispielsweise Derivate der Diaminostilbendisulfonsäure beziehungsweise deren Alkalimetallsalze enthalten. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls oder 4-(4-Chlorstyryl)-4'-(2-sulfostyryl)-diphenyls. Auch Gemische der vorgenannten Aufheller können verwendet werden.

Zu den geeigneten Schauminhibitoren gehören beispielsweise Organopolysiloxane und deren Gemische mit mikrofeiner, gegebenenfalls silanierter Kieselsäure sowie Pa-raffinwachse und deren Gemische mit silanierter Kieselsäure oder Bisfettsäure-alkylendiamiden. Mit Vorteilen werden auch Gemische aus verschiedenen Schaum-inhibitoren verwendet, zum Beispiel solche aus Silikonen, Paraffinen oder Wachsen. Vorzugsweise sind die Schauminhibitoren, insbesondere Silikonund/oder Paraffin-haltige Schauminhibitoren, an eine granulare, in Wasser lösliche beziehungsweise dispergierbare Trägersubstanz gebunden. Insbesondere sind dabei Mischungen aus Paraffinwachsen und Bistearylethylendiamiden bevorzugt.

Zusätzlich können die Verbrauchsprodukte auch Komponenten enthalten, welche die Öl- und Fettauswaschbarkeit aus Textilien positiv beeinflussen, sogenannte soil release-Wirkstoffe. Dieser Effekt wird besonders deutlich, wenn ein Textil verschmutzt wird, das bereits vorher mehrfach mit einem Waschmittel, das diese öl- und fettlösende Komponente enthält, gewaschen wurde. Zu den bevorzugten öl- und fettlösenden Komponenten zählen beispielsweise nicht-ionische Celluloseether wie Methylcellulose und Methylhydroxypropylcellulose mit einem Anteil an Methoxyl-Gruppen von 15 bis 30 Gew.-% und an Hydroxypropoxyl-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten mit monomeren und/oder polymeren Diolen, insbesondere Polymere aus Ethylenterephthalaten und/oder Polyethylenglykolterephthalaten oder anionisch und/oder nichtionisch modifizierten Derivaten von diesen.

Die Verbrauchsprodukte können auch Farbübertragungsinhibitoren, vorzugsweise in Mengen von 0,1 Gew.-% bis 2 Gew.-%, insbesondere 0,1 Gew.-% bis 1 Gew.-%, enthalten, die in einer bevorzugten Ausgestaltung der Erfindung Polymere aus Vinylpyrrolidon, Vinylimidazol, Vinylpyridin-N-Oxid oder Copolymere aus diesen sind. Brauchbar sind sowohl Polyvinylpyrrolidone mit Molgewichten von 15 000 bis 50 000 wie auch Polyvinylpyrrolidone mit Molgewichten über 1 000 000, insbesondere von 1 500 000 bis 4 000 000, N-Vinylimidazol/N-Vinylpyrrolidon-Copolymere, Potyvinyloxazolidone, Copolymere auf Basis von Vinylmonomeren und Carbonsäureamiden, pyrrolidongruppenhaltige Polyester und Polyamide, gepfropfte Polyamidoamine und Polyethylenimine, Polymere mit Amidgruppen aus sekundären Aminen, Polyamin-N-Oxid-Polymere, Polyvinylalkohole und Copolymere auf Basis von Acrylamidoalkenylsulfonsäuren.

Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt werden Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf das Verbrauchsprodukt, eingesetzt.

Zu den in den erfindungsgemäßen Verbrauchsprodukten, insbesondere wenn sie in flüssiger oder pastöser Form vorliegen, verwendbaren organischen Lösungsmitteln gehören Alkohole mit 1 bis 4 C-Atomen, insbesondere Methanol, Ethanol, Isopropanol und tert.-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel sind in den erfindungsgemäßen Verbrauchsprodukten, wie z.B. Waschmitteln, vorzugsweise in Mengen von nicht über 30 Gew.-%, insbesondere von 6 Gew.-% bis 20 Gew.-%, vorhanden.

Zur Einstellung eines gewünschten, sich durch die Mischung der übrigen Komponenten nicht von selbst ergebenden pH-Werts können die erfindungsgemäßen Verbrauchsproduktee system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, enthalten. Derartige pH-Regulatoren sind in den erfindungsgemäßen Verbrauchsprodukten vorzugsweise nicht über 20 Gew.-%, insbesondere von 1,2 Gew.-% bis 17 Gew.-%, enthalten.

Die Herstellung fester erfindungsgemäßer Verbrauchsprodukte bereitet keine Schwierigkeiten und kann im Prinzip bekannter Weise, zum Beispiel durch Sprühtrocknen oder Granulation, erfolgen, wobei Persauerstoffverbindung und Bleichkatalysator gegebenenfalls später zugesetzt werden. Die erfindungsgemäße Parfümzusammensetzungen sowie ggf. weitere Riechstoffe werden vorzugsweise zum Ende der Herstellung auf das Verbrauchsprodukt aufgebracht. Zur Herstellung erfindungsgemäßer Verbrauchsprodukte mit erhöhtem Schüttgewicht, insbesondere im Bereich von 650 g/l bis 950 g/l, ist ein einen Extrusionsschritt aufweisendes Verfahren bevorzugt. Die Herstellung flüssiger erfindungsgemäßer Verbrauchsprodukte bereitet ebenfalls keine Schwierigkeiten und kann ebenfalls in bekannter Weise erfolgen, wobei die erfindungsgemäße Parfümzusammensetzung sowie ggf. weitere Riechstoffe vorzugsweise zum Ende der Herstellung in das Verbrauchsprodukt, z.B. Waschmittel, eingebracht werden.

Gemäß einer bevorzugten Ausführungsform kann die erfindungsgemäße Lehre dazu eingesetzt werden, den Parfümanteil in Verbrauchsprodukten wie Wasch-, Reinigungs- und Körperpflegemitteln signifikant herabzusetzen. Dadurch ist es möglich, parfümierte Produkte auch für solche besonders empfindlichen Konsumenten anzubieten, die normal parfümierte Produkte aufgrund spezieller Unverträglichkeiten und Irritationen nur eingeschränkt oder überhaupt nicht verwenden können. In diesem Zusammenhang sind vor allem Hautpflegeprodukte und Deodorantien, aber auch Waschmittel, wie z.B. Handwaschmittel zu nennen.

Ein bevorzugtes erfindungsgemäßes Verbrauchsprodukt ist ein festes, insbesondere pulverförmiges Waschmittel, das neben der erfindungsgemäßen Parfümzusammensetzung vorzugsweise Komponenten enthalten kann, die z.B. ausgewählt sind aus den folgenden:
- Aniontenside, wie vorzugsweise Alkylbenzolsulfonat, Alkylsulfat, z.B. in Mengen von vorzugsweise 5-30 Gew.-%
- Nichtionische Tenside, wie vorzugsweise Fettalkoholpolyglycolether, Alkylpolyglucosid, Fettsäureglucamid z.B. in Mengen von vorzugsweise 0,5-15 Gew.-%
- Gerüststoffe, wie z.B. Zeolith, Polycarboxylat, Natriumcitrat, in Mengen von z.B. 0-70 Gew.-% , vorteilhafterweise 5-60 Gew.-%, vorzugsweise 10-55 Gew.-%, insbesondere 15-40 Gew.-%,
- Alkalien, wie z.B. Natriumcarbonat, in Mengen von z.B. 0-35 Gew.-% vorteilhafterweise 1-30 Gew.-%, vorzugsweise 2-25 Gew.-%, insbesondere 5-20 Gew.-%,
- Bleichmittel, wie z.B. Natriumperborat, Natriumpercarbonat, in Mengen von z.B. 0-30 Gew.-% vorteilhafterweise 5-25 Gew.-%, vorzugsweise 10-20 Gew.-%,
- Korrosionsinhibitoren, z.B. Natriumsilicat, in Mengen von z.B. 0-10 Gew.-%, vorteilhafterweise 1-6 Gew.-%, vorzugsweise 2-5 Gew.-%, insbesondere 3-4 Gew.-%,
- Stabilisatoren, z.B. Phosphonate, vorteilhafterweise 0-1 Gew.-%,
- Schauminhibitor, z.B. Seife, Siliconöle, Paraffine vorteilhafterweise 0-4 Gew.-%, vorzugsweise 0,1-3 Gew.-%, insbesondere 0,2-1 Gew.-%,
- Enzyme, z.B. Proteasen, Amylasen, Cellulasen, Lipasen, vorteilhafterweise 0-2 Gew.-%, vorzugsweise 0,2-1 Gew.-%, insbesondere 0,3-0,8 Gew.-%,
- Vergrauungsinhibitor, z.B. Carboxymethylcellulose, vorteilhaftenweise 0-1 Gew.-%,
- Verfärbungsinhibitor, z.B. Polyvinylpyrrolidon-Derivate, z.B. 0-2 Gew.-%,
- Stellmittel, z.B. Natriumsulfat, vorteilhafterweise 0-20 Gew.-%,
- Optische Aufheller, z.B. Stilben-Derivat, Biphenyl-Derivat, vorteilhafterweise 0-0,4 Gew.-%, insbesondere 0,1-0,3 Gew.-%,
- ggf. weitere Riechstoffe
- ggf. Wasser
- ggf.Seife
- ggf.Bleichaktivatoren
- ggf.Cellulosderivate
- ggf.Schmutzabweiser,
Gew.-% jeweils bezogen auf das gesamte Mittel.

In einer anderen bevorzugten Ausführungsform der Erfindung liegt das Verbrauchsprodukt in flüssiger Form vor, vorzugsweise in Gelform. Bevorzugte flüssige Wasch- oder Reinigungsmittel haben Wassergehalte von z.B. 10-95 Gew.-%, vorzugsweise 20-80 Gew.-% und insbesondere 30-70 Gew.-%, bezogen auf das gesamte Mittel. Im Falle von flüssigen Konzentraten kann der Wassergehalt auch besonders gering sein, z.B. ≤ 30 Gew.-%, vorzugsweise ≤ 20 Gew.-%, insbesondere 15 Gew.-% betragen, Ges.-% jeweils bezogen auf das gesamte Mittel. Die flüssigen Verbrauchsprodukte können auch nichtwässrige Lösungsmittel enthalten.

Ein bevorzugtes erfindungsgemäßes Verbrauchsprodukt ist ein flüssiges, insbesondere gelförmiges Waschmittel, das neben der erfindungsgemäßen Parfümzusammensetzung vorzugsweise Komponenten enthalten kann, die z.B. ausgewählt sind aus den folgenden:
- Aniontenside, wie vorzugsweise Alkylbenzolsulfonat, Alkylsulfat, z.B. in Mengen von vorzugsweise 5-40 Gew.-%
- Nichtionische Tenside, wie vorzugsweise Fettalkoholpolyglycolether, Alkylpolyglucosid, Fettsäureglucamid z.B. in Mengen von vorzugsweise 0,5-25 Gew.-%
- Gerüststoffe, wie z.B. Zeolith, Polycarboxylat, Natriumcitrat, vorteilhafterweise 0-15 Gew.-%, vorzugsweise 0,01-10 Gew.-%, insbesondere 0,1-5 Gew.-%,
- Schauminhibitor, z.B. Seife, Siliconöle, Paraffine, in Mengen von z.B. 0-10 Gew.-%, vorteilhafterweise 0,1-4 Gew.-%, vorzugsweise 0,2-2 Gew.-%, insbesondere 1-3 Gew.-%,
- Enzyme, z.B. Proteasen, Amylasen, Cellulasen, Lipasen, in Mengen von z.B. 0-3 Gew.-%, vorteilhafterweise 0,1-2 Gew.-%, vorzugsweise 0,2-1 Gew.-%, insbesondere 0,3-0,8 Gew.-%,
- Optische Aufheller, z.B. Stilben-Derivat, Biphenyl-Derivat, in Mengen von z.B. 0-1 Gew.-%, vorteilhafterweise 0,1-0,3 Gew.-%, insbesondere 0,1-0,4 Gew.-%,
- ggf. weitere Riechstoffe
- ggf. Stabilisatoren,
- Wasser
- ggf. Seife, in Mengen von z.B. 0-25 Gew.-%, vorteilhafterweise 1-20 Gew.-%, vorzugsweise 2-15 Gew.-%, insbesondere 5-10 Gew.-%,
- ggf. Lösungsmittel (vorzugsweise Alkohole), vorteilhafterweise 0-25 Gew.-%, vorzugsweise 1-20 Gew.-%, insbesondere 2-15 Gew.-%, Gew.-% jeweils bezogen auf das gesamte Mittel.

Ein bevorzugtes erfindungsgemäßes Verbrauchsprodukt ist ein flüssiger Weichspüler, der neben der erfindungsgemäßen Parfümzusammensetzung vorzugsweise Komponenten enthalten kann, die ausgewählt sind aus den folgenden:
- Kationische Tenside, wie insbesondere Esterquats, z.B. in Mengen von 5-30 Gew.-%,
- Cotenside, wie z.B. Glycerolmonostearat, Stearinsäure, Fettalkohole, Fettalkoholethoxylate, z.B. in Mengen von 0-5 Gew.-%, vorzugsweise 0,1-4 Gew.-%,
- Emulgatoren, wie z.B. Fettaminethoxylate, z.B. in Mengen von 0-4 Gew.-%, vorzugsweise 0,1-3 Gew.-%,
- ggf. weitere Riechstoffe
- ggf. Farbstoffe, vorzugsweise im ppm-Bereich
- ggf. Stabilisatoren, vorzugsweise im ppm-Bereich
- Lösemittel, wie insbesondere Wasser, z.B. in Mengen von 60-90 Gew.-%,
Gew.-% jeweils bezogen auf das gesamte Mittel.

### Beispiel

Beispiel A: Es wurde eine übliche Riechstoffmischung in üblicher Weise durch Aufsprühen in ein vorher parfümfreies Universalpulverwaschmittel, umfassend 15 Gew.-% Tenside (anionische und nichtionische Tenside sowie Seife), > 30 Gew.-% Builder (Soda, Silicate, Zeolite, Polymere), 5 Gew.-% Bleichmittel auf Sauerstoffbasis eingearbeitet. Das resultierende Waschmittel wurde als Waschmittel A bezeichnet und enthielt insgesamt 0,3 Gew.-% Riechstoffe.

Beispiel B: Es wurde eine Riechstoffmischung, die sich von der Riechstoffmischung in Beispiel A nur dadurch unterschied, dass sie zusätzlich Methylserinol enthielt, ebenfalls in üblicher Weise durch Aufsprühen in ein Universalpulverwaschmittel eingearbeitet. Das resultierende Waschmittel wurde als Waschmittel B bezeichnet und unterschied sich von dem aus Beispiel A nur durch die Anwesenheit von Methylserinol. Die Riechstoffmischung gemäß Beispiel B wurde hergestellt, indem bei Raumtemperatur 1 Gew.-Teil des Methylserinols in die ansonsten fertige Riechstoffmischung (100 Gew.-Teile) gegeben wurde. Dann wurde mit einem Magnetrührer gerührt, bis sich das Methylserinol aufgelöst hatte.

Mit den Waschmitteln A und B wurden dann Lagertests und Anwendungstests durchgeführt.

### Lagertests:

Für den Lagertest wurden die beiden Waschmittel jeweils in getrennten Klimakammem bei 40°C für vier Wochen gelagert. Danach wurde der Duft der Waschmittel von 7 Parfumeuren geprüft und die Veränderung des Dufteindrucks gegenüber frischer Ware bewertet. Es wurde die qualitative Veränderung des Dufteindruckes von jedem Parfumeur auf einer Skala mit einer Note von 1 bis 5 bewertet (5 = keine Veränderung des Duftes erkennbar; 4 = Veränderung des.Duftes gerade noch bemerkbar; 3 = Veränderung des Duftes sehr gering, aber vom Parfumeur schnell erkennbar; 2 = geringe Veränderung des Duftes; 1 = deutliche Veränderung des Duftes).

Nach der Lagerung bewerteten die Parfumeure das Waschmittel A mit der durchschnittlichen Note 1,9 (gemittelter Wert aller Parfumeure über 2 Testdurchläufe). Das Waschmittel B wurde dagegen mit einer 4,0 bewertet (gemittelter Wert aller Parfumeure bei 2 Testdurchläufen).

### Anwendungstests:

Die Waschmittel A und B wurden nach vierwöchiger Lagerung bei 40°C für Waschversuche eingesetzt. Es wurden jeweils weiße Baumwollunterhemden mit den Waschmitteln in üblicher Dosierung gewaschen. Es wurde jeweils bei 40°C eine normale Maschinenwäsche durchgeführt. Die gewaschene Wäsche wurde sowohl im feuchten wie im trockenen Zustand von 7 Parfumeuren hinsichtlich des Duftes geprüft. Dabei bewerteten die Parfumeure auf einer Skala von 1 bis 10 (10 = exzellente Leistung, entspricht außergewöhnlichen Ansprüchen; 9 = hervorragende Leistung; 8 = sehr gute Leistung; 7 = gute Leistung, die erheblich über den durchschnittlichen Anforderungen liegt; 6 = noch gute Leistung, die noch über den durchschnittlichen Anforderungen liegt; 5 = voll befriedigend, entspricht durchschnittlichen Anforderungen voll; 4 = befriedigend, entspricht noch den durchschnittlichen Anforderungen; 3 = ausreichend, trotz deutlicher Mängel ; 2 = mangelhaft, erhebliche Mängel, Anforderungen werden verfehlt; 1 = ungenügend, eine völlig unbrauchbare Leistung).

Die Wäsche, die mit dem Waschmittel A gewaschen wurde, beurteilt im feuchten Zustand, erhielt die durchschnittliche Note 5,0 (gemittelter Wert aller Parfumeure über 2 Testdurchläufe). Die Wäsche, die mit dem Waschmittel A gewaschen wurde, beurteilt im getrockneten Zustand (nach Trocknung an der Leine), erhielt die durchschnittliche Note 3,9 (gemittelter Wert aller Parfumeure über 2 Testdurchläufe).

Die Wäsche, die mit dem Waschmittel B gewaschen wurde, beurteilt im feuchten Zustand, erhielt die durchschnittliche Note 7,2 (gemittelter Wert aller Parfumeure über 2 Testdurchläufe). Die Wäsche, die mit dem Waschmittel B gewaschen wurde, beurteilt im getrockneten Zustand (nach Trocknung an der Leine), erhielt die durchschnittliche Note 7,1 (gemittelter Wert aller Parfumeure über 2 Testdurchläufe).

## Patentansprüche

1. Parfümzusammensetzung, umfassend Aminoalkohol(e) gemäß Formel (I)
HO-CR1R2-CR3R4-NHR5, (I)
wobei in dieser Formel die Reste R1, R2, R3, R4 sowie R5, jeweils unabhängig voneinander, für Wasserstoff oder (ggf. substituierte) Kohlenwasserstoffreste (insbesondere Hydroxyalkylreste) stehen,
sowie > 10 Gew.-% Riechstoffe.

2. Zusammensetzung gemäß Anspruch 1,**dadurch gekennzeichnet, dass** sie Aminoalkohol gemäß der Formel (II) umfasst 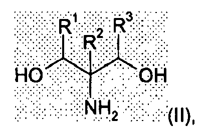 wobei in dieser Formel die Reste R¹, R² sowie R³, jeweils unabhängig voneinander, für Wasserstoff oder (ggf. substituierte) Kohlenwasserstoffreste (insbesondere Hydroxyalkylreste) stehen, insbesondere 2-Aminopropan-1,3-diol, 2-Amino-2-(hydroxymethyl)propan-1,3-diol, 2-Amino-2-Methylpropan-1,3-diol, 2-Amino-2-Ethyl-propan-1,3-diol und/oder 1-Phenyl-2-Amino-propan-1,3-diol umfasst.

3. Zusammensetzung gemäß einem der Ansprüche 1 oder 2, **dadurch gekennzeichnet, dass** sie > 60 Gew.-%, vorzugsweise > 70 Gew.-%, vorteilhafterweise > 80 Gew.-%, insbesondere > 90 Gew.-% Riechstoffe enthält.

4. Zusammensetzung nach einem der Ansprüche 1 bis 3, **dadurch gekennzeichnet, dass** mehr als 1 Gew.-%, in vorteilhafter Weise mehr als 5 Gew.-%, insbesondere mehr als 10 Gew.-% der enthaltenen Riechstoffe aus solchen Riechstoffen ausgewählt sind, welche eine Aldehydfunktion ( RCH=O), eine Keto-Gruppe ( RR'C=O ), eine Hydroxyfunktion (-OH) und/oder Gruppen mit isolierten Doppelbindungen tragen, wobei sich die Angabe Gew.-% auf die Gesamtmenge der im Mittel enthaltenen Riechstoffe bezieht.

5. Zusammensetzung gemäß einem der Ansprüche 1 bis 4, **dadurch gekennzeichnet, dass** als Riechstoff zumindest ein Duftaldehyd enthalten ist, ausgewählt aus Adoxal, Anisaldehyd, Cymal, Ethylvanillin, Florhydral, Helional, Heliotropin, Hydroxycitronellal, Koavon, Lauraldehyd, Lyral, Methylnonylacetaldehyd, P. T. Bucinal, Phenylacetaldehyd, Undecylenaldehyd, Vanillin, 2,6,10-Trimethyl-9-undecenal, 3-Dodecen-1-al, alpha-n-Amylzimtaldehyd, 4-Methoxybenzaldehyd, Benzaldehyd, 3-(4-tert-Butylphenyl)-propanal, 2-Methyl-3-(para-methoxyphenyl)propanal, 2-Methyl-4-(2,6,6-timethyl-2(1)-cyclohexen-1-yl)butanal, 3-Phenyl-2-propenal, cis-/trans-3,7-Dimethyl- 2,6-octadien-1-al, 3,7-Dimethyl-6-octen-1-al, [(3,7-Dimethyl-6-octenyl)oxy]acetaldehyd, 4-Isopropylbenzylaldehyd, 1,2,3,4,5,6,7,8-Octahydro-8,8-dmethyl-2-naphthatdehyd, 2,4-Dimethyl-3-cyclo-hexen-1-carboxaldehyd, 2-Methyl-3-(isopropylphenyl)propanal, 1-Decanal, Decylaldehyd, 2,6-Dimethyl-5-heptenal, 4-(Tricyclo[5.2.1.0(2,6)]-decyliden-8)-butanal, Octahydro-4,7-methan-1 H-indencarboxaldehyd, 3-Ethoxy-4-hydroxybenzaldehyd, para-Ethyl-alpha,alphadimethylhydrozimtaldehyd, alpha-Methyl-3,4-(methylendioxy)-hydrozimtaldehyd, 3,4-Methylendioxybenzaldehyd, alpha-n-Hexylzimtaldehyd, m-Cymen-7-carboxaldehyd, alpha-Methylphenylacetaldehyd, 7-Hydroxy-3,7-dimethyloctanal, Undecenal, 2,4,6-Trimethyl-3-cyclohexen-1-carboxaldehyd, 4-(3)(4-Methyl-3-pentenyl)-3-cyclohexencarboxaldehyd, 1-Dodecanal, 2,4-Dimethylcyclohexen-3-carboxaldehyd, 4-(4-Hydroxy-4-methylpentyl)-3-cylohexen-1-carboxaldehyd, 7-Methoxy-3,7-dimethyloctan-1-al 2-Methylundecanal, 2-Methyldecanal, 1-Nonanal, 1-Octanal, 2,6,10-Trimethyl-5,9-undecadienal, 2-Methyl- 3-(4-tert-butyl)propanal, Dihydrozimtaldehyd, 1-Methyl-4-(4-methyl-3-pentenyl)-3-cyclohexen-1-carboxaldehyd, 5- oder 6-Methoxyhexahydro-4,7-methanindan-1- oder - 2-carboxaldehyd, 3,7-Dimethyloctan-1-al, 1-Undecanal, 10-Undecen-1-al, 4-Hydroxy-3-methoxybenzaldehyd, 1-Methyl-3-(4-methylpentyl)-3-cyclohexencarboxaldehyd, 7-Hydroxy-3,7-dimethyloctanal, trans-4-Decenal, 2,6-Nonadienal, para-Tolylacetaldehyd, 4-Methylphenylacetaldehyd, 2-Methyl-4-(2,6,6-trimethyl-1-cyclohexen-1-yl)-2-butenal, ortho-Methoxyzimtaldehyd, 3,5,6-Trimethyl-3-cyclohexencarboxaldehyd, 3,7-Dimethyl-2-methylen-6-octenal, Phenoxyacetaldehyd, 5,9-Dimethyl-4,8-decadienal, Päonienaldehyd (6,10-Dimethyl-3-oxa-5,9-undecadien-1-al), Hexahydro-4,7-methanindan-1-carboxaldehyd, 2-Methyloctanal, alpha-Methyl-4-(1-methylethyl)benzolacetaldehyd, 6,6-Dimethyl-2-norpinen-2-propionaldehyd, para-Methylphenoxyacetaldehyd, 2-Methyl-3-phenyl-2-propen- 1-al 3,5,5-Trimethylhexanal, Hexahydro-8,8-dimethyl-2-naphthaldehyd, 3-Propylbicyclo[2.2.1]-hept-5-en-2-carbaldehyd, 9-Decenal, 3-Methyl-5-phenyl-1-pentanal, Methylnonylacetaldehyd, Hexanal, trans-2-Hexenal, 1-p-Menthen-q-carboxaldehyd oder Mischungen davon.

6. Zusammensetzung gemäß einem der Ansprüche 1 bis 5, **dadurch gekennzeichnet, dass** als Riechstoff zumindest ein Duftketon enthalten ist, ausgewählt aus Buccoxim; iso-Jasmon; Methyl-beta-naphthylketon; Moschusindanon; Tona-lid/Musk plus; alpha-Damascon, .beta-Damascon, delta-Damascon, iso-Damascon, Damascenon, Damarose, Methyldihydrojasmonat, Menthon, Carvon, Kampfer, Fenchon, alpha-Ionon, beta-Ionon, gamma-Methyl genannt Ionon, Fleuramon, Dihydrojasmon, cis-Jasmon, iso-E-Super®, Methylcedrenylketon oder Methylcedry-lon, Acetophenon, Methylacetophenon, para-Methoxyacetophenon, Methyl-beta-naphtylketon, Benzylaceton, Benzophenon, para-Hydroxyphenylbutanon, Sellerie-Keton oder Livescone, 6-Isopropyldecahydro-2-naphton, Dimethyloctenon, Frescomenthe, 4-(1-Ethoxyvinyl)-3,3,5,5-tetramethylcyclohexanon, Methylheptenon, 2-(2-(4-Methyl-3-cyclohexen-1-yl)propyl)cyclopentanon, 1-(p-Menthen-6(2)yl)-1-propanon, 4-(4-Hydroxy-3-methoxyphenyl)-2-butanon, 2-Acetyl-3,3-dimethylnorbornan, 6,7-Dihydro-1,1,2,3,3-pentamethyl-4(5H)in-danon, 4-Damascol, Dulcinyl oder Cassion, Gelson, HexaIon, Isocyclemon E, Methylcyclocitron, Methyllaven-delketon, Orivon, para-tertiärem Butylcyclohexanon, Verdon, Delphon, Muscon, Neobutenon, Plicaton, Velouton, 2,4,4,7-Tetramethyl-oct-6-en-3-on, Tetrameran oder Mischungen davon.

7. Zusammensetzung gemäß einem der Ansprüche 1 bis 6, **dadurch gekennzeichnet, dass** der Aminoalkohol in Mengen von 0,001 bis 30 Gew.-%, vorzugsweise 0,1 bis 10 Gew.-%, insbesondere 1 bis 5 Gew.-% enthalten ist.

8. Zusammensetzung gemäß einem der Ansprüche 1 bis 7, **dadurch gekennzeichnet, dass** sie < 15 Gew.-%, vorzugsweise < 5 Gew.-%, insbesondere < 1 Gew.-% Tenside umfasst.

9. Verfahren zur Stabilisierung von Parfümölen, **dadurch gekennzeichnet, dass** 0,001 bis 30 Gew.-%, vorzugsweise 0,1 bis 10 Gew.-%, insbesondere 1 bis 5 Gew.-% Aminoalkohol gemäß Formel (I)
HO-CR1 R2-CR3R4-NHR5, (I)
wobei in dieser Formel die Reste R1, R2, R3, R4 sowie R5, jeweils unabhängig voneinander, für Wasserstoff oder (ggf. substituierte) Kohlenwasserstoffreste (insbesondere Hydroxyalkylreste) stehen,
in das Parfümöl gegeben werden.

10. Verfahren zur Herstellung parfümierter Verbrauchsprodukte (insbesondere Wasch- oder Reinigungsmittel, Kosmetika, Luftverbesserer, Klebstoffe), **dadurch gekennzeichnet, dass** eine Parfümzusammensetzung nach einem der Ansprüche 1 bis 8 zugegeben wird.

11. Verwendung einer Parfümzusammensetzung nach einem der Ansprüche 1 bis 8 bei der Herstellung parfümierter Wasch-, Reinigungs- oder Pflegemittels zur Verlängerung der Duftwirkung des Wasch-, Reinigungs- oder Pflegemittels und/oder zur Erzielung eines lange anhaltenden Frischegeruches bei der Anwendung des Wasch-, Reinigungs- oder Pflegemittels.

## Claims

1. A perfume composition comprising amino alcohol(s) according to formula (I)
HO-CR1R2-CR3R4-NHR5 (I),
where in said formula the residues R1, R2, R3, R4, and R5, mutually independently in each case, denote hydrogen or (optionally substituted) hydrocarbon residues (in particular hydroxyalkyl residues) as well as > 10 wt% fragrances.

2. The composition according to Claim 1, **characterized in that** it comprises amino alcohol according to formula (II) 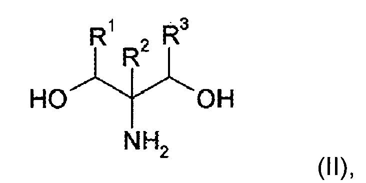 where in said formula the residues R¹, R², and R³, mutually independently in each case, denote hydrogen or (optionally substituted) hydrocarbon residues (in particular hydroxyalkyl residues), in particular 2-aminopropane-1,3-diol, 2-amino-2-(hydroxymethyl)propane-1,3-diol, 2-amino-2-methylpropane-1,3-diol, 2-amino-2-ethylpropane-1,3-diol, and/or 1-phenyl-2-aminopropane-1,3-diol.

3. The composition according to one of Claims 1 or 2, **characterized in that** it contains > 60 wt%, preferably > 70 wt%, advantageously > 80 wt%, in particular > 90 wt% fragrances.

4. The composition according to one of Claims 1 to 3, **characterized in that** more than 1 wt%, advantageously more than 5 wt%, in particular more than 10 wt% of the fragrances contained are selected from those fragrances which carry an aldehyde function (RCH=O), a keto group (RR'C=O), a hydroxy function (-OH), and/or groups having isolated double bonds, the "wt%" indication referring to the total quantity of fragrances contained in the agent.

5. The composition according to one of Claims 1 to 4, **characterized in that** at least one scent aldehyde selected from adoxal, anisaldehyde, cymal, ethyl vanillin, florhydral, helional, heliotropin, hydroxycitronellal, koavon, lauraldehyde, lyral, methylnonylacetaldehyde, P-T-bucinal, phenylacetaldehyde, undecylenealdehyde, vanillin, 2,6,10-trimethyl-9-undecenal, 3-dodecen-1-al, alpha-n-amylcinnamaldehyde, 4-methoxybenzaldehyde, benzaldehyde, 3-(4-tert-butylphenyl)propanal, 2-methyl-3-(para-methoxyphenyl)propanal, 2-methyl-4-(2,6,6-trimethyl-2(1)-cyclohexen-1-yl)butanal, 3-phenyl-2-propenal, cis-/trans-3,7-dimethyl-2,6-octadien-1-al, 3,7-dimethyl-6-octen-1-al, [(3,7-dimethyl-6-octenyl)oxy]acetaldehyde, 4-isopropylbenzylaldehyde, 1,2,3,4,5,6,7,8-octahydro-8,8-dimethyl-2-naphthaldehyde, 2,4-dimethyl-3-cyclohexene-1-carboxaldehyde, 2-methyl-3-(isopropylphenyl)propanal, 1-decanal, decylaldehyde, 2,6-dimethyl-5-heptenal, 4-(tricyclo[5.2.1.0(2,6)]-decylidene-8)-butanal, octahydro-4,7-methane-1H-indenecarboxaldehyde, 3-ethoxy-4-hydroxybenzaldehyde, para-ethylalpha,alpha-dimethylhydrocinnamaldehyde, alpha-methyl-3,4-(methylenedioxy)hydrocinnamaldehyde, 3,4-methylenedioxybenzaldehyde, alpha-n-hexylcinnamaldehyde, m-cymene-7-carboxaldehyde, alpha-methylphenylacetaldehyde, 7-hydroxy-3,7-dimethyloctanal, undecenal, 2,4,6-trimethyl-3-cyclohexene-1-carboxaldehyde, 4-(3)(4-methyl-3-pentenyl)-3-cyclohexenecarboxaldehyde, 1-dodecanal, 2,4-dimethylcyclohexene-3-carboxaldehyde, 4-(4-hydroxy-4-methylpentyl)-3-cylohexene-1-carboxaldehyde, 7-methoxy-3,7-dimethyloctan-1-al, 2-methylundecanal, 2-methyldecanal, 1-nonanal, 1-octanal, 2,6,10-trimethyl-5,9-undecadienal, 2-methyl-3-(4-tert-butyl)propanal, dihydrocinnamaldehyde, 1-methyl-4-(4-methyl-3-pentenyl)-3-cyclohexene-1-carboxaldehyde, 5- or 6-methoxyhexahydro-4,7-methane indane-1- or -2-carboxaldehyde, 3,7-dimethyloctan-1-al, 1-undecanal, 10-undecen-1-al, 4-hydroxy-3-methoxybenzaldehyde, 1-methyl-3-(4-methylpentyl)-3-cyclohexenecarboxaldehyde, 7-hydroxy-3,7-dimethyloctanal, trans-4-decenal, 2,6-nonadienal, para-tolylacetaldehyde, 4-methylphenylacetaldehyde, 2-methyl-4-(2,6,6-trimethyl-1-cyclohexen-1-yl)-2-butenal, ortho-methoxycinnamaldehyde, 3,5,6-trimethyl-3-cyclohexenecarboxaldehyde, 3,7-dimethyl-2-methylene-6-octenal, phenoxyacetaldehyde, 5,9-dimethyl-4,8-decadienal, peony aldehyde (6,10-dimethyl-3-oxa-5,9-undecadien-1-al), hexahydro-4,7-methane indane-1-carboxaldehyde, 2-methyloctanal, alpha-methyl-4-(1-methylethyl)benzeneacetaldehyde, 6,6-dimethyl-2-norpinene-2-propionaldehyde, para-methylphenoxyacetaldehyde, 2-methyl-3-phenyl-2-propen-1-al, 3,5,5-trimethylhexanal, hexahydro-8,8-dimethyl-2-naphthaldehyde, 3-propylbicyclo[2.2.1]-hept-5-ene-2-carbaldehyde, 9-decenal, 3-methyl-5-phenyl-1-pentanal, methylnonylacetaldehyde, hexanal, trans-2-hexenal, 1-p-menthene-q-carboxaldehyde, or mixtures thereof, is contained as a fragrance.

6. The composition according to one of Claims 1 to 5, **characterized in that** at least one scent ketone, selected from Buccoxime; isojasmone; methylbeta-naphthylketone; musk indanone; Tonalid/Musk plus; alphadamascone, beta-damascone, delta-damascone, isodamascone, damascenone, damarose, methyldihydrojasmonate, menthone, carvone, camphor, fenchone, alpha-ionone, beta-ionone, gamma-methyl (socalled) ionone, fleuramone, dihydrojasmone, cis-jasmone, iso-E-Super^{®}, methylcedrenyl ketone or methyl cedrylone, acetophenone, methylacetophenone, para-methoxyacetophenone, methyl-beta-naphtyl ketone, benzylacetone, benzophenone, para-hydroxyphenylbutanone, celery ketone or livescone, 6-isopropyldecahydro-2-naphthone, dimethyloctenone, frescomenthe, 4-(1-ethoxyvinyl)-3,3,5,5-tetramethylcyclohexanone, methylheptenone, 2-(2-(4-methyl-3-cyclohexen-1-yl)propyl)cyclopentanone, 1-(p-menthen-6(2)yl)-1-propanone, 4-(4-hydroxy-3-methoxyphenyl)-2-butanone, 2-acetyl-3,3-dimethylnorbornane, 6,7-dihydro-1,1,2,3,3-pentamethyl-4(5H)indanone, 4-damascol, dulcinyl or cassione, gelsone, hexalone, isocyclemone E, methylcyclocitrone, methyl lavender ketone, orivone, para-tertiary butylcyclohexanone, verdone, delphone, muscone, neobutenone, plicatone, veloutone, 2,4,4,7-tetramethyloct-6-en-3-one, tetrameran, or mixtures thereof, is contained as a fragrance.

7. The composition according to one of Claims 1 to 6, **characterized in that** the amino alcohol is contained in quantities from 0.001 to 30 wt%, preferably 0.1 to 10 wt%, in particular 1 to 5 wt%.

8. The composition according to one of Claims 1 to 7, **characterized in that** it comprises < 15 wt%, preferably < 5 wt%, in particular < 1 wt% surfactants.

9. A method for stabilizing perfume oils, **characterized in that** 0.001 to 30 wt%, preferably 0.1 to 10 wt%, in particular 1 to 5 wt% amino alcohol according to formula (I)
HO-CR1 R2-CR3R4-NHR5 (I),
where in said formula the residues R1, R2, R3, R4, and R5, mutually independently in each case, denote hydrogen or (optionally substituted) hydrocarbon residues (in particular hydroxyalkyl residues),
is added to the perfume oil.

10. A method for manufacturing perfumed consumer products (in particular washing or cleaning agents, cosmetics, air fresheners, adhesives), **characterized in that** a perfume composition according to one of Claims 1 to 8 is added.

11. Use of a perfume composition according to one of Claims 1 to 8 in the manufacture of perfumed washing, cleaning, or care-providing agents in order to extend the scenting effect of the washing, cleaning, or care-providing agent and/or to achieve a long-lasting fresh odor upon utilization of the washing, cleaning, or care-providing agent.

## Revendications

1. Composition de parfum, comprenant un/des aminoalcool(s) selon la formule (I)
HO-CR1 R2-CR3R4-NHR5, (I)
dans laquelle les résidus R1, R2, R3, R4 ainsi que R5 représentent, indépendamment l'un de l'autre, hydrogène ou des résidus hydrocarbonés (le cas échéant substitués) (notamment des résidus hydroxyalkyle),
ainsi que > 10 % en poids de substances odorantes.

2. Composition selon la revendication 1, **caractérisée en ce que** qu'elle comprend de l'aminoalcool selon la formule (II) 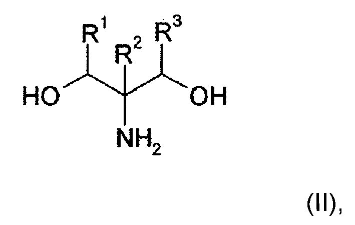 dans laquelle les résidus R¹, R² ainsi que R³ représentent, indépendamment l'un de l'autre, hydrogène ou des résidus hydrocarbonés (le cas échéant substitués) (notamment des résidus hydroxyalkyle), et comprend plus particulièrement le 2-aminopropan-1,3-diol, le 2-amino-2-(hydroxyméthyl)propan-1,3-diol, le 2-amino-2-méthyl-propan-1,3-diol, le 2-amino-2-éthyl-propan-1,3-diol et/ou le 1-phényl-2-amino-propan-1,3-diol

3. Composition selon l'une des revendications 1 ou 2, **caractérisée en ce qu'**elle contient > 60 % en poids, de préférence > 70 % en poids, avantageusement > 80 % en poids, notamment > 90 % en poids de substances odorantes.

4. Composition selon l'une des revendications 1 à 3, **caractérisée en ce que** plus de 1 % en poids, avantageusement plus de 5 % en poids, notamment plus de 10 % en poids des substances odorantes qu'elle contient sont choisies parmi les substances odorantes pourvues d'une fonction aldéhyde (RCH=O), d'un groupe céto ( RR'C=O ), d'une fonction hydroxyle (-OH) et/ou de groupes comportant des doubles liaisons isolées, les pourcentages en poids étant exprimés par rapport à la quantité globale des substances odorantes contenues dans l'agent.

5. Composition selon l'une des revendications 1 à 4, **caractérisée en ce qu'**elle contient, en tant que substance odorante, au moins un aldéhyde de parfumerie choisi parmi l'adoxal, l'anisaldéhyde, le cymal, l'éthylvanillin, le florhydral, l'hélional, l'héliotropine, l'hydroxycitronellal, la koavone, le lauraldéhyde, le lyral, le méthylnonylacétaldéhyde, le P-T-bucinal, le phenylacétaldéhyde, l'undécylènealdéhyde, la vanilline, le 2,6,10-triméthyl-9-undécenal, le 3-dodécen-1-al, l'alpha-n-amylcinnamaldéhyde, le 4-méthoxybenzaldéhyde, le benzaldéhyde, le 3-(4-tert-butylphenyl)-propanal, le 2-méthyl-3-(paraméthoxyphenyl)propanal, le 2-méthyl-4-(2,6,6-triméthyl-2(1)-cyclohexène-1-yl)butanal, le 3-phenyl-2-propenal, le cis-/trans-3,7-diméthyl- 2,6-octadiène-1-al, le 3,7-diméthyl-6-octène-1-al, le [(3,7-diméthyl-6-octényl)oxy]acétaldéhyde, le 4-isopropylbenzylaldéhyde, le 1,2,3,4,5,6,7,8-octahydro-8,8-diméthyl-2-naphthaldéhyde, le 2,4-diméthyl-3-cyclo-hexène-1-carboxaldéhyde, le 2-méthyl-3-(isopropylphényl)propanal, le 1-décanal, le décylaldéhyde, le 2,6-diméthyl-5-heptenal, le 4-(tricyclo[5.2.1.0(2,6)]-décyliden-8)-butanal, l'octahydro-4,7-méthan-1H-indènecarboxaldéhyde, le 3-éthoxy-4-hydroxybenzaldéhyde, le para-éthyl-alpha,alpha-diméthylhydrocinnamaldéhyde, l'alpha-méthyl-3,4-(méthylènedioxy)- hydrocinnamaldéhyde, le 3,4-méthylènedioxybenzaldéhyde, l'alpha-n-hexylcinnamaldéhyde, le m-cymène-7-carboxaldéhyde, l'alpha-méthylphénylacétaldéhyde, le 7-hydroxy-3,7-diméthyloctanal, l'undécenal, le 2,4,6-triméthyl-3-cyclohexène-1-carboxaldéhyde, le 4-(3)(4-méthyl-3-pentényl)-3-cyclohexènecarboxaldéhyde, le 1-dodécanal, le 2,4-diméthylcyclohexène-3-carboxaldéhyde, le 4-(4-hydroxy-4-méthylpentyl)-3-cylohexène-1-carboxaldéhyde, le 7-méthoxy-3,7-diméthyloctan-1-al, le 2-méthylundécanal, le 2-méthyldécanal, le 1-nonanal, le 1-octanal, le 2,6,10-triméthyl-5,9-undécadienal, le 2-méthyl-3-(4-tert-butyl)propanal, le dihydrocinnamaldéhyde, le 1-méthyl-4-(4-méthyl-3-pentényl)-3-cyclohexène-1-carboxaldéhyde, le 5- ou 6-méthoxyhexahydro-4,7-méthane-indane-1- ou - 2-carboxaldéhyde, le 3,7-diméthyloctan-1-al, le 1-undécanal, le 10-undécène-1-al, le 4-hydroxy-3-méthoxybenzaldéhyde, le 1-méthyl-3-(4-méthylpentyl)-3-cyclohexènecarboxaldéhyde, le 7-hydroxy-3,7-diméthyloctanal, le trans-4-décénal, le 2,6-nonadiénal, le para-tolylacétaldéhyde, le 4-méthylphénylacétaldéhyde, le 2-méthyl-4-(2,6,6-triméthyl-1-cyclohexène-1-yl)-2-buténal, le ortho-méthoxycinnamaldéhyde, le 3,5,6-triméthyl-3-cyclohexènecarboxaldéhyde, le 3,7-diméthyl-2-méthylène-6-octénal, le phénoxyacétaldéhyde, le 5,9-diméthyl-4,8-décadiénal, l'aldéhyde de paeonia (6,10-diméthyl-3-oxa-5,9-undécadiène-1-al), l'hexahydro-4,7-méthane-indane-1-carboxaldéhyde, le 2-méthyloctanal, l'alpha-méthyl-4-(1-méthyléthyl)benzèneacétaldéhyde, le 6,6-diméthyl-2-norpinène-2-propionaldéhyde, le paraméthylphénoxyacétaldéhyde, le 2-méthyl-3-phényl-2-propène-1-al, le 3,5,5-triméthylhexanal, le hexahydro-8,8-diméthyl-2-naphthaldéhyde, le 3-propylbicyclo[2.2.1]-hept-5-ène-2-carbaldéhyde, le 9-décénal, le 3-méthyl-5-phenyl-1-pentanal, le méthylnonylacétaldéhyde, l'hexanal, le trans-2-hexénal, le 1-p-menthène-q-carboxaldéhyde ou parmi leurs mélanges.

6. Composition selon l'une des revendications 1 à 5, **caractérisée en ce qu'**elle contient, en tant que substance odorante, une cétone de parfumerie choisie parmi le buccoxime ; l'iso-jasmone ; la méthyl-bêta-naphthylcétone ; l'indanone musquée ; le Tone-lid/musk plus ; l'alpha-damascone, la bêta-damascone, la delta-damascone, l'iso-damascone, la damascénone, le damarose, le méthyldihydrojasmonate, la menthone, la carvone, le camphre, la fenchone, l'alpha-ionone, la bêta-ionone, gamma-méthyl appelé ionone, la fleuramone, la dihydrojasmone, la cis-jasmone, l'iso-E-Super®, la méthylcédrénylcétone ou méthylcédrylone, l'acétophénone, la méthylacétophénone, la para-méthoxyacétophénone, la méthyl-bêta-naphthylcétone, la benzylacétone, la benzophénone, la para-hydroxyphénylbutanone, la cétone de céléri ou livescone, la 6-isopropyldécahydro-2-naphthone, la diméthylocténone, la frescomenthe, la 4-(1-éthoxyvinyl)-3,3,5,5-tétraméthylcyclohexanone, la méthylhepténone, la 2-(2-(4-méthyl-3-cyclohexène-1-yl)propyl)cyclopentanone, la 1-(p-menthène-6(2)yl)-1-propanone, la 4-(4-hydroxy-3-méthoxyphényl)-2-butanone, le 2-acétyl-3,3-diméthylnorbornane, la 6,7-dihydro-1,1,2,3,3-pentaméthyl-4(5H)indanone, le 4-damascol, le dulcinyl ou la cassione, la gelsone, l'hexalone, l'isocyclémone E, la méthylcyclocitrone, la méthylcétone de lavande, l'orivone, la para-tert.-butylcyclohexanone, la verdone, la delphone, la muscone, la néobuténone, la plicatone, la veloutone, la 2,4,4,7-tétraméthyl-oct-6-ène-3-on, le tétramérane ou parmi leurs mélanges.

7. Composition selon l'une des revendications 1 à 6, **caractérisée en ce qu'**elle contient ledit aminoalcool dans des quantités comprises entre 0,001 et 30 % en poids, de préférence entre 0,1 et 10 % en poids, notamment entre1 et 5 % en poids.

8. Composition selon l'une des revendications 1 à 7, **caractérisée en ce qu'**elle comprend < 15 % en poids, de préférence < 5 % en poids, notamment < 1 % en poids d'agents tensioactifs.

9. Procédé de stabilisation de concentrés de parfum, **caractérisé en ce que** l'on ajoute audit concentré de parfum 0,001 à 30 % en poids, de préférence 0,1 à 10 % en poids, notamment 1 à 5 % en poids d'aminoalcool selon la formule (I)
HO-CR1 R2-CR3R4-NHR5, (I)
dans laquelle les résidus R1, R2, R3, R4 ainsi que R5 représentent, indépendamment l'un de l'autre, hydrogène ou des résidus hydrocarbonés (le cas échéant substitués) (notamment des résidus hydroxyalkyle).

10. Procédé de fabrication de produits de grande consommation parfumés (s'agissant notamment d'agents de lavage ou de nettoyage, de produits cosmétiques, de parfums d'ambiance, d'agents adhésifs), **caractérisé en ce que** l'on ajoute une composition de parfum selon l'une des revendications 1 à 8.

11. Utilisation d'une composition de parfum selon l'une des revendications 1 à 8 lors de la fabrication d'agents de lavage, de nettoyage ou d'entretien parfumés, pour prolonger l'effet odoriférant de l'agent de lavage, de nettoyage ou d'entretien et/ou pour obtenir une odeur fraîche de longue durée lors de l'utilisation de l'agent de lavage, de nettoyage ou d'entretien.
